# EUROPÄISCHE PATENTANMELDUNG

(11) **EP 3 563 890 A1**
(43) Veröffentlichungstag der Anmeldung: **06.11.2019**
(21) Anmeldenummer: 19175183.3
(22) Anmeldetag: 04.06.2009
(51) Int. Cl.: A61M 1/36, A61M 1/16, A61M 1/34

(54) **ORGANIZER ZUM LÖSBAREN AUFNEHMEN VON KOMPONENTEN VON BLUTSCHLAUCHSÄTZEN UND VERFAHREN ZUM HERSTELLEN SOWIE ZUM VORBEREITEN DESSELBEN**

(30) Priorität: 05.06.2008 DE 102008026916
(62) Teilanmeldung aus: 09757296.0
(71) Anmelder: Fresenius Medical Care Deutschland GmbH, 61352 Bad Homburg (DE)
(72) Erfinder: KLEWINGHAUS, Jürgen, 61440 Oberursel (DE); HERRENBAUER, Michael, 71636 Ludwigsburg (DE); NACHBAUR-STURM, Christine, 53783 Eitorf (DE); DI RIENZO, Giovanni, Hemel Hempstead, HP1 1HZ (GB); REITER, Reinhold, I-26013 Crema (IT)
(74) Vertreter: Bobbert & Partner Patentanwälte PartmbB

(57) **Zusammenfassung**

Die vorliegende Erfindung betrifft einen Organizer (1) zum lösbaren Aufnehmen von Komponenten (2, 3, 4, 5, 6, 35) von Blutschlauchsätzen für unterschiedliche extrakorporale Blutbehandlungsoptionen, insbesondere bei Dialyseverfahren, mittels einer Blutbehandlungsvorrichtung sowie Verfahren zum Herstellen eines erfindungsgemäßen Organizers (1).

## Beschreibung

Die vorliegende Erfindung betrifft einen Organizer mit den Merkmalen des Oberbegriffs des Anspruchs 1. Sie betrifft ferner ein Verfahren zum Herstellen eines Organizers gemäß dem Oberbegriff des Anspruchs 14 und ein hierin beschriebenes Verfahren zum Vorbereiten des Organizers.

Aus der Praxis sind Vorrichtungen zur extrakorporalen Blutbehandlung, insbesondere zur Dialysebehandlung, bekannt, mittels welcher wahlweise unterschiedliche Behandlungsverfahren bzw. Behandlungsoptionen ausgeführt werden können. Dabei ist für unterschiedliche Behandlungsoptionen ein jeweils unterschiedlicher Umfang an Blutschlauchsätzen (Disposables) und zugehörigen Komponenten, wie z. B. Schlauchverbindungen und dergleichen, erforderlich.

Aus dem Stand der Technik sind Zusammenstellungen von Komponenten zum Bilden von Blutschläuchen bekannt, aus welchen sich Blutschläuche bzw. Behandlungssets für unterschiedliche Behandlungsoptionen zusammenstellen lassen. Solche Zusammenstellungen, wie sie bspw. aus der EP 1 159 977 B1 bekannt sind, können auf sogenannten "Organizern" oder "Trays" übersichtlich und für die Ankoppel-Gegebenheiten der verwendeten Blutbehandlungsvorrichtung passend vorgehalten werden. Je nach durchzuführender Behandlungsoption können die hierzu benötigten Blutschlauchsatz-Komponenten entsprechend konnektiert werden. Die für die ausgewählte Behandlungsoption nicht benötigten Blutschlauchsatz-Komponenten verbleiben ungenutzt am Organizer.

Ein hiermit verbundener Nachteil besteht darin, dass die Vielzahl der am Organizer vorliegenden Komponenten sowie deren vielfältige Verbindungsmöglichkeiten miteinander verwirrend auf das Klinikpersonal wirkt, weshalb es regelmäßig und insbesondere unter Zeitdruck zu fehlerhafter Auswahl und Konnektierung von Komponenten kommt. Zudem erfordert das Vorbereiten eines einsatzfähigen Blutschlauchsatzes aus den am Organizer vorhandenen Komponenten für die ausgewählte Behandlungsoption Erfahrung und ist überdies zeitaufwändig, da oft mehrere Schlauchsätze oder Schlauchleitungen gewissenhaft verfolgt werden müssen, um eine fehlerhafte Verbindung und Verwechslungen zu vermeiden und dabei eine große Anzahl ähnlich aussehender Komponenten im Blick behalten werden muss.

Es ist Aufgabe der vorliegenden Erfindung, einen weiteren Organizer bzw. ein weiteres Tray anzugeben.

Die erfindungsgemäße Aufgabe wird durch einen Organizer mit den Merkmalen des Anspruchs 1 gelöst.

So wird erfindungsgemäß ein Organizer zum lösbaren Aufnehmen von Komponenten von Blutschläuchen für unterschiedliche extrakorporale Blutbehandlungsoptionen, insbesondere bei Dialyseverfahren, mit einer Blutbehandlungsvorrichtung, wie bspw. einer Dialysevorrichtung, vorgeschlagen. Dabei ist der Organizer geeignet und vorbereitet, Komponenten aufzunehmen, mittels welcher im Gebrauch des Organizers wahlweise wenigstens ein erster Blutschlauchsatz für eine erste Blutbehandlungsoption oder ein zweiter Blutschlauchsatz für eine zweite - von der ersten unabhängigen - Blutbehandlungsoption einer Gruppe mit mehreren, wenigstens zwei, möglichen Blutschlauchsätzen, ausgestaltet werden können.

Unter einer "extrakorporalen Blutbehandlungsoption" wird erfindungsgemäß jedes Blutbehandlungsverfahren, zu welchem die eingesetzte Blutbehandlungsvorrichtung geeignet ist, verstanden. Zu diesen Behandlungsoptionen zählen beim Beispiel der Dialyse unter anderem die Hämofiltration, die Hämodialyse, die Hämodiafiltration, die Slow-Continuous-Ultrafiltration, die Membrane-Plasma-Separation sowie die hochvolumige Hämofiltration.

Unter einem "Blutschlauchsatz" wird erfindungsgemäß jeweils ein einsatzfertiger Blutschlauchsatz (oder ein Blutschlauch-Behandlungsset) verstanden, welcher eine oder mehrere Komponenten aufweist. Dabei kann unter einem Blutschlauchsatz die Summe aller am Organizer vorliegenden Komponenten, welche jeweils bei einer bestimmten Behandlungsoption zum Einsatz kommt, verstanden werden.

Die für eine Blutbehandlungsoption nicht benötigten Komponenten des erfindungsgemäßen Organizers können vor Beginn der Behandlung einfach und sicher entfernt und schließlich verworfen werden. Hierzu können Mittel zum sicheren Abtrennen und Herausnehmen von nicht benötigten Komponenten bzw. Baugruppen vorgesehen sein. Diese Mittel können eine Einrichtung zum lösbaren Befestigen von Blutschlauchsatzabschnitten oder Komponenten am Organizer umfassen. Die Mittel können dergestalt sein, dass ein individuelles Lösen mancher Komponenten möglich ist, ohne dass andere, zur Behandlung benötigte Komponenten, ebenfalls gelöst werden. Somit können manche Komponenten unabhängig von anderen gelöst und ggf. verworfen werden.

Die Erfindung zeichnet sich vorteilhaft durch ein hohes Maß an erreichbarer Sicherheit beim Verbinden der Behandlungsvorrichtung mit dem Blutschlauchssatz aus, da eine Verwechslungsgefahr von Komponenten bzw. Fehlkonnektierung zum Vorbereiten eines Blutschlauchsatzes für eine ausgewählte der möglichen unterschiedlichen Behandlungsoptionen aufgrund der Möglichkeit, nicht benötigte Komponenten zu verwerfen, weitestgehend ausgeschlossen ist.

Dies erlaubt, dass nur die tatsächlich benötigten Komponenten beim Primen und Spülen mit Lösung befüllt werden müssen, was vorteilhaft zum Einsparen von Primern und Lösungen beiträgt.

Ferner kann bei einem Organizer, welcher geeignet und vorbereitet ist zur Aufnahme von Komponenten zum Herstellen verschiedener, getrennt voneinander verwendbarer Blutschlauchsätze, deren Konnektion mit der Behandlungsvorrichtung einfach und übersichtlich und damit zeitunaufwändig erfolgen. Die Anordnung der einzelnen Komponenten zur Behandlungsvorrichtung ist mittels des Organizers optimierbar.

Die am Organizer vorliegenden Komponenten können ferner unterschiedliche Anschlusselemente aufweisen, welche mit der Behandlungsvorrichtung jeweils für jene Behandlungsoption verbunden werden können, für welche sie bestimmt sind, für andere hingegen nicht. Auch dies dient der Erhöhung der Sicherheit bei Einsatz und beim Konnektieren von Blutschlauchsätzen.

Die Vorbereitung eines Blutschlauchsatzes ist bei Einsatz des erfindungsgemäßen Organizers nicht mehr nur besonders erfahrenem Personal vorbehalten, sondern kann auch durch weniger erfahrene Mitarbeiter erfolgen.

Die vorliegende Erfindung erlaubt ferner, den Organizer bei unverminderter Sicherheit und schneller Handhabung - gegenüber einem Bereitstellen eines einzeln vorliegenden Blutschlauchsatzes - mit allen Komponenten für verschiedene Blutschlauchsätze am Behandlungsort bereitzustellen, so dass durch nur einen Organizer sichergestellt werden kann, dass tatsächlich alle Komponenten für einen Blutschlauchsatz für jede mögliche oder gewünschte Blutbehandlungsoption vor Ort präsent ist.

Ein weiterer Vorteil des erfindungsgemäßen Organizers besteht darin, dass sein Einsatz die Lagerhaltung für Komponenten und für Blutschlauchsätze stark vereinfachen kann. Die Vielzahl der zu verwaltenden Artikel kann auf einen einzigen, universellen Artikel, nämlich den Organizer selbst, reduziert werden. Zwar müssen hierzu regelmäßig Komponenten des Organizers verworfen werden, jedoch sind die jeweils erzielbaren Kostenersparnisse angesichts der vereinfachten Lagerhaltung größer als die Mehrkosten für das Verwerfen unbenötigter Komponenten.

Vorteilhafte Weiterentwicklungen des erfindungsgemäßen Organizers sind jeweils Gegenstand der Unteransprüche.

So wird in einer bevorzugten erfindungsgemäßen Ausführungsform ein einstückiger Organizer vorgeschlagen. Hierdurch unterscheidet sich der erfindungsgemäße Organizer bspw. von dem in der EP 0 914 048 B1 in deren Fig. 12 dargestellten Organizer. Letzterer ist zum übersichtlichen Anordnen einer Vielzahl von Komponenten wie Schläuchen für einen oder mehrere Blutschlauchsätze geeignet. Durch seinen rahmenartigen Aufbau mit einer oberen oder unteren Rahmenhälfte ist es in der Praxis jedoch kaum möglich, einzelne Schlauchabschnitte zu entnehmen, ohne dass sich andere Schlauchabschnitte ebenfalls vom Organizer lösen oder in Unordnung geraten. Diesem Nachteil wird durch die einstückige Ausgestaltung des erfindungsgemäßen Organizers in dieser Ausführungsform der Erfindung abgeholfen.

Unter einer "einstückigen" Ausgestaltung wird erfindungsgemäß auch eine solche verstanden, bei welcher nach bspw. einem Gieß- oder Spritzvorgang zum Herstellen des Hauptkörpers des Organizers diesem weitere Bauelemente hinzugefügt werden. Erfindungsrelevant ist im Zusammenhang mit "einstückig" lediglich, dass der Organizer dieser erfindungsgemäßen Ausführungsform nicht etwa - wie der oben beschriebene Rahmenaufbau des Standes der Technik - zur Entnahme von nicht benötigten Blutschlauchsatzkomponenten geöffnet werden muss. Die Auswahl der für eine vorgesehene Blutbehandlungsoption erforderlichen Komponenten und ein Verwerfen der übrigen Komponenten ist somit auch auf kleinstem Raum und ohne das Risiko, dabei eine Unordnung innerhalb der Gruppe der Blutschlauchsätze bzw. -komponenten zu verursachen, vorteilhaft möglich.

Mittels des erfindungsgemäßen Organizers ist es vorteilhaft möglich, gezielt bestimmte Komponenten, Schlauchabschnitte oder ganze Schlauchsätze vom Organizer zu entfernen, ohne weitere Komponenten unbeabsichtigterweise ebenfalls zu lösen. Ein unbeabsichtigtes Lösen von Komponenten wird somit verhindert.

Derselbe Vorteil wie bei der einstückigen Ausgestaltung ist auch dann erreichbar, wenn der Organizer von einer freiliegenden - im Sinne einer frei zugänglichen - Seite mit Blutschlauchsatzkomponenten bestückbar ausgestaltet ist. Auch dies soll als "einstückig" verstanden werden.

In einer weiter bevorzugten Ausführungsform weist der Organizer wenigstens eine Befestigungseinrichtung zum lösbaren Befestigen von Blutschlauchsatzkomponenten - eines oder mehrerer Blutschlauchsätze - auf. Ein unbeabsichtigtes Lösen von Schlauchabschnitten oder dergleichen vom Organizer kann durch gezielten Einsatz der Befestigungseinrichtung vorteilhaft vermieden werden. Hierdurch lassen sich fehlerträchtige Situationen vermeiden. Zudem trägt die Befestigungseinrichtung dazu bei, bestimmte Schlauchabschnitte gezielt zu lösen, ohne unbeabsichtigterweise weitere Komponenten ebenfalls vom Organizer zu trennen.

Eine besonders einfache und kostengünstige Ausgestaltung der Befestigungseinrichtung des erfindungsgemäßen Organizers weist in einer bevorzugten Ausführungsform wenigstens einen Aufnahmeabschnitt zum lösbaren Aufnehmen wenigstens einer Komponente am Organizer auf, wobei diese mit einer Vertiefung zum Aufnehmen der Komponente entlang eines Abschnitts eines Umfangs der Komponente ausgestaltet sein kann. Die Befestigungseinrichtung weist in dieser Ausführungsform ferner eine Lasche oder Klappe auf, mittels welcher die Komponente im Aufnahmeabschnitt lösbar gesichert werden kann. Dies kann insbesondere ein Verschließen der Vertiefung des Aufnahmeabschnitts und insbesondere deren reversibles Verschließen umfassen.

Diese Ausgestaltung der Befestigungseinrichtung weist gegenüber aus dem Stand der Technik bekannten Befestigungseinrichtungen, wie sie bspw. als Clip aus der US 6,298,525 B1 bekannt sind, Verbesserungen auf. Solche Clips bzw. bekannte Befestigungseinrichtungen allgemein weisen den Nachteil auf, dass die durch sie auf die Komponente des Blutschlauchsatzes zu deren Fixierung ausgeübte Spannung in Folge von Werkstoffrelaxationen mit der Zeit abgebaut wird. Solche Relaxationen können bspw. bedingt durch die Dauer oder die Umstände der Lagerung oder bereits die Sterilisation der Befestigungseinrichtung oder des die Befestigungseinrichtung tragenden Organizers auftreten. Infolge der Relaxation können sich Komponenten unbeabsichtigt aus der Befestigung lösen, was insbesondere bei Vorliegen einer Vielzahl von Komponenten für unterschiedliche Behandlungsoptionen wiederum zu unübersichtlichen und fehlerträchtigen Situationen führen kann.

Das Vorsehen der Lasche, mittels welcher die Komponente oder mehrere Komponenten im Aufnahmeabschnitt oder in mehreren Aufnahmeabschnitten lösbar gesichert werden, erzwingt zum Lösen der Komponente einen bewusst durchzuführenden Arbeitsschritt. Dieser Arbeitsschritt kann in einem Hochklappen, einem Wegschwenken oder dergleichen der Lasche oder Klappe bestehen. Ein unbeabsichtigtes Lösen des Schlauchabschnitts wie bspw. aus dem oben beschriebenen Clip oder einem aus dem Stand der Technik ebenfalls bekannten, rillenförmigen Schlitz, wie u. a. in der US 2003/0132352 A1 offenbart, kann erfindungsgemäß vermieden werden. Zumindest wird die Wahrscheinlichkeit des Auftretens merklich gesenkt.

Dabei kann der Aufnahmeabschnitt insbesondere mit einer Vertiefung ausgestaltet sein, in welcher Abschnitte der Komponente, insbesondere entlang eines Umfangsabschnitts hiervon, aufgenommen werden können. Die Lasche kann vorgesehen sein, um die Vertiefung entlang ihres Umfanges zu verschließen, d. h., um ihren Umfang form- und/ oder kraftschlüssig zu vervollständigen.

Die Lasche kann - wie bei einer weiter bevorzugten Ausführungsform vorgesehen - elastisch ausgestaltet sein. Dies erlaubt ein einfaches Hochklappen oder - biegen der Lasche zur Entnahme der Komponente aus dem zugehörigen Aufnahmeabschnitt. Eine solche elastische Ausgestaltung sichert das Verbleiben der Komponente im Aufnahmeabschnitt. Einem versehentlichen Öffnen der Lasche, welches auch noch unbemerkt bleiben könnte, ist aufgrund der Elastizität der Lasche und der hiermit einhergehenden Rückstellwirkung vorteilhaft vorgebeugt.

In einer wiederum weiter bevorzugten Ausführungsform weist die Befestigungseinrichtung wenigstens einen Abschnitt dergestalt auf, dass ein Anheben der Lasche mittels des Fingers eines Benutzers erleichtert ist. Hierzu kann der Abschnitt geneigt sein und hierdurch ein Hintergreifen des Fingers zwischen Lasche und Organizer erlauben. Dieselbe vorteilhafte Wirkung kann durch das Vorsehen eines Überstandes der Lasche, welcher wiederum einfach mit dem Finger zu greifen ist, erzielt oder verstärkt werden. Die Neigung des Abschnitts kann sich insbesondere auf eine Haupterstreckungsebene des Organizers beziehen. Die positive oder negative Neigung des Abschnitts kann bezogen auf die Haupterstreckungsebene des Organizers bevorzugt einen Winkel von 20-80°, besonders bevorzugt von 30-70°, ganz besonders bevorzugt von 40-60° sowie jeden Zwischenwert dieser Bereiche annehmen.

Die Lasche der Befestigungseinrichtung des Organizers kann in einer weiter bevorzugten Ausführungsform ferner verschwenkbar angeordnet sein. Dabei kann eine optionale elastische Ausgestaltung der Lasche wiederum ein automatisches Zurückschwenken in eine Sicherungsstellung bewirken, unter Erzielung der oben diskutierten Vorteile. Ferner kann ein Einrasten der Lasche in bspw. einem Abschnitt der Befestigungseinrichtung zu deren Sicherung gegen ein unbeabsichtigtes Verschwenken aus der Sicherungsstellung heraus vorgesehen sein.

In einer wiederum weiter bevorzugten Ausführungsform weist ein beliebiger wie oben beschriebener Organizer Komponenten zum Ausgestalten wenigstens eines Blutschlauchsatzes auf. Bei einem derart ausgestatteten Organizer können wenigstens eine Befestigungseinrichtung und wenigstens eine Blutschlauchsatzkomponente derart in ihren Dimensionen aufeinander abgestimmt sein, dass die Blutschlauchsatzkomponente in der Befestigungseinrichtung - zusätzlich oder alternativ zu anderen Sicherungs- und Befestigungseinrichtungen - verklemmbar ist. Auch hierdurch wird ein unbeabsichtigtes und ungewolltes Lösen von Komponenten vom Organizer vorteilhaft verhindert.

Der Organizer kann in jeder Ausführungsform aus Polystyrol gefertigt sein oder ein solches aufweisen. Dabei kann der Organizer u. a. mittels Thermoformen hergestellt sein.

In einer weiter bevorzugten Ausführungsform des erfindungsgemäßen Organizers wird ein darin eingelegter Schlauch nur oder vorwiegend nur durch Formschluss gehalten. Bei einer solchen Ausführungsform wird der Schlauch nur bei seinem Einlegen und/oder bei seiner Entnahme elastisch zusammengedrückt, und das auch nur kurzfristig, nämlich dann, wenn er die Engstelle durchtritt, um in einen zu seiner Aufnahme vorgesehenen Kanal eingelegt zu werden. Einmal eingelegt wird der Schlauch bei dieser Ausführungsform vorteilhaft nicht oder nur unwesentlich elastisch verformt. Eine solche Ausführungsform hat den weiteren Vorteil, dass während des Sterilisierens (z.B. beim Dampfsterilisieren) des Organizers mit eingelegten Komponenten im Bereich des Clips keine oder nur unwesentliche plastische Verformungen des Schlauchs durch Relaxation (mit der Zeit abnehmende Spannungen bei gleich bleibender Verformung) auftreten.

Die erfindungsgemäße Aufgabe wird ferner gelöst durch ein Verfahren zum Herstellen eines Organizers, insbesondere nach einem der vorangegangenen Ausführungen, mit den Merkmalen des Anspruchs 14. Zudem wird die erfindungsgemäße Aufgabe gelöst durch ein Verfahren zum Vorbereiten eines, insbesondere erfindungsgemäßen, Organizers zum lösbaren Aufnehmen von Komponenten von Blutschlauchsätzen für unterschiedliche extrakorporale Blutbehandlungsoptionen mit dem Schritt des Entfernens von Komponenten und/oder Blutschlauchsätzen, die für eine ausgewählte Blutbehandlungsoption nicht benötigt werden. Da mit dem erfindungsgemäßen Verfahren ungeschmälert die oben im Zusammenhang mit dem erfindungsgemäßen Organizer diskutierten Vorteile erzielbar sind, wird zur Vermeidung von Wiederholungen an dieser Stelle explizit auf deren obige Diskussion verwiesen.

Beim erfindungsgemäßen Organizer kann es möglich sein, während einer Unterbrechung der Blutbehandlung Teile bzw. Komponenten des Blutschlauchsatzes oder den gesamten Blutschlauchsatz gegen neue Disposables auszutauschen. Ein solcher Austausch von Teilen kann z. B. aufgrund einer Blutkoagulation im Filter erforderlich werden. Der an der Maschine angehängte Organizer würde in diesem Fall weiterverwendet werden.

In manchen erfindungsgemäßen, beispielhaften Ausführungsformen umfasst das erfindungsgemäße Verfahren den Schritt des einstückigen Ausgestaltens des Organizers.

In einigen erfindungsgemäßen, beispielhaften Ausführungsformen umfasst das erfindungsgemäße Verfahren das, insbesondere lösbare, Aufnehmen von Komponenten von Blutschlauchsätzen für eine Blutbehandlung mit unterschiedlichen extrakorporalen Blutbehandlungsoptionen, insbesondere für Dialyseverfahren. mittels einer Blutbehandlungsvorrichtung.

Die vorliegende Erfindung wird im Folgenden anhand der beigefügten Zeichnungen exemplarisch beschrieben. Dabei bezeichnen gleiche Bezugszeichen gleiche Elemente. In der Zeichnung gilt:
- **Fig. 1**: zeigt einen exemplarischen erfindungsgemäßen Organizer;
- **Fig. 2**: zeigt ein mögliches Blockschaltbild für eine Hämodiafiltration;
- **Fig. 3**: zeigt in Tabellenform jeweils die für eine Behandlungsoption verwendeten Blutschlauchsätze sowie ggf. entnommene S chlauchsatzkomponenten;
- **Fig. 4**: zeigt eine Zugangsleitung vom Patienten zum Filter, welche auch in der Fig. 1 gezeigt ist;
- **Fig. 5**: zeigt eine Substituatleitung, wie sie auch in Fig. 1 gezeigt ist;
- **Fig. 6**: zeigt einen nicht mit Blutschlauchsatzkomponenten bestückten erfindungsgemäßen Organizer in einer Draufsicht;
- **Fig. 7**: zeigt eine Befestigungseinrichtung mit einem Aufnahmeabschnitt zum lösbaren Aufnehmen eines Abschnitts eines Blutschlauchs in einer ersten Ausführungsform hiervon;
- **Fig. 8**: zeigt die Befestigungseinrichtung der Fig. 7;
- **Fig. 9**: zeigt die Befestigungseinrichtung der Figs. 7 und 8;
- **Fig. 10**: zeigt die aus den Figs. 7, 8 und 9 bekannte Befestigungseinrichtung;
- **Fig. 11**: zeigt eine Befestigungseinrichtung in einer zweiten Ausführungsform hiervon;
- **Fig. 12**: zeigt eine dritte Ausführungsform der Befestigungseinrichtung für einen Organizer mit zwei Laschen zur Sicherung von drei Aufnahmeabschnitten;
- **Fig. 13**: zeigt eine vierte Ausführungsform der erfindungsgemäßen Befestigungseinrichtung in einer perspektivischen Ansicht;
- **Fig. 14**: zeigt die Befestigungseinrichtung der Fig. 13 in einer Draufsicht;
- **Fig. 15**: zeigt die Befestigungseinrichtung der Fig. 13 und 14 in einer weiteren perspektivischen Ansicht; und
- **Fig. 16**: zeigt die Befestigungseinrichtung der Fig. 13 bis 15 in einer Seitenansicht.

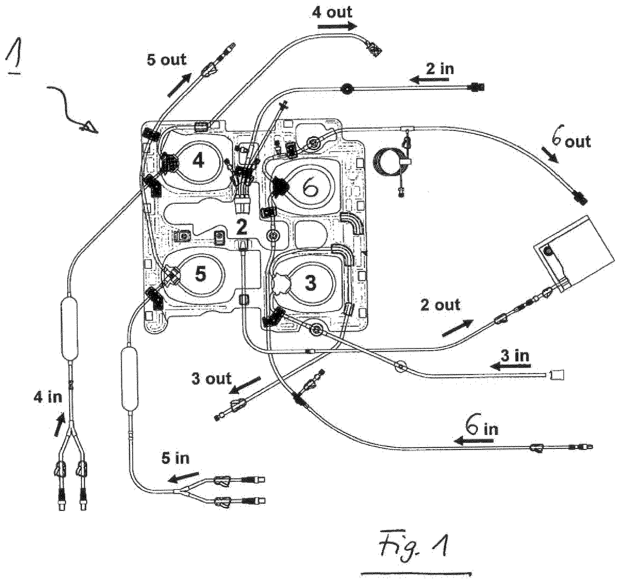

**Fig. 1** zeigt schematisch vereinfacht einen exemplarisch bestückten erfindungsgemäßen Organizer 1, welcher bestückt ist mit einer Rückgabeleitung 2 von einem nicht dargestellten Filter zum Patienten, einer Filtratleitung 3 vom Filter zu einem nicht dargestellten Filtratbeutel, einer Dialysatleitung 4 für Dialysierflüssigkeit oder Austauschflüssigkeit von einem nicht dargestellten Lösungsbeutel zum Filter oder Anschlussstück, einer Substituatleitung 5 für Austauschflüssigkeit vom Lösungsbeutel zum Anschlussstück in einer Zugangsleitung 6 vom Patienten zum Filter oder der Rückgabeleitung 2.

Dabei umfasst die Rückgabeleitung 2 einen Filterkonnektor 2in sowie einen Patientenkonnektor 2out mit Spülbeutel. Die Filtratleitung 3 umfasst einen Filterkonnektor 3in sowie einen Konnektor 3out für den Filtratbeutel. Die Dialysatleitung 4 umfasst einen Konnektor 4in für den Lösungsbeutel und einen Filterkonnektor 4out oder für ein Anschlussstück in der Zugangs- oder Rückgabeleitung. Die Substituatleitung 5 umfasst einen Konnektor 5in für den Lösungsbeutel sowie einen Konnektor 5out für das Anschlussstück in der Zugangs- oder Rückgabeleitung. Die Zugangsleitung 6 vom Patienten zum Filter umfasst einen Patientenkonnektor 6in sowie einen Filterkonnektor 6out.

Der Organizer 1 kann dabei Aufhängeösen X, Y aufweisen, die wie in Fig. 1 gezeigt in Form von Aussparungen ausgeführt oder anderweitig ausgestaltet sein können. Mittels dieser kann der Organizer 1 an zwei an der Behandlungsvorrichtung (nicht gezeigt in Fig. 1) vorgesehenen Haken oder Knöpfen angehängt werden. Während der Behandlung verbleibt der Organizer 1 mit den an ihm verbliebenen Komponenten an der Behandlungsvorrichtung. Die weiter unten beschriebenen Befestigungsvorrichtungen des Organizers dienen u. a. während der Behandlung zum Führen oder Halten der Schläuche bzw. Komponenten, so dass zu diesem Zweck keine maschinenenseitigen Halterungen vorgesehen werden müssen. Erst nach der Behandlung wird der Organizer 1 von der Behandlungsvorrichtung abgehängt. Vorzugsweise ist der Organizer 1 so gestaltet, dass eine vorherige Abnahme des Organizers (ohne daran befindliche Komponenten) von der Behandlungsvorrichtung nicht oder zumindest nicht versehentlich möglich ist.

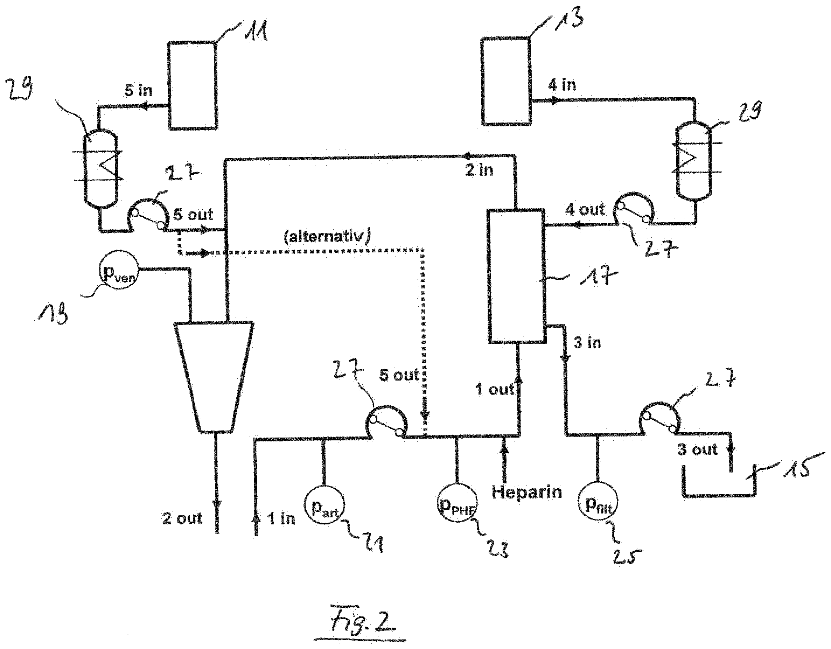

**Fig. 2** zeigt ein mögliches Blockschaltbild für eine Hämodiafiltration mit allen auch in Fig. 1 gezeigten Komponenten. Ferner zeigt Fig. 2 einen Substituatbeutel 11, einen Dialysatbeutel 13, einen Filtratbeutel 15 sowie eine Dialyseeinrichtung 17. In Fig. 2 sind ferner Druckaufnehmer 19, 21, 23 und 25 sowie weitere dem Fachmann bekannte Komponenten für extrakorporale Blutkreisläufe wie Pumpsegmente 27 und Heizbeutel 29 dargestellt.

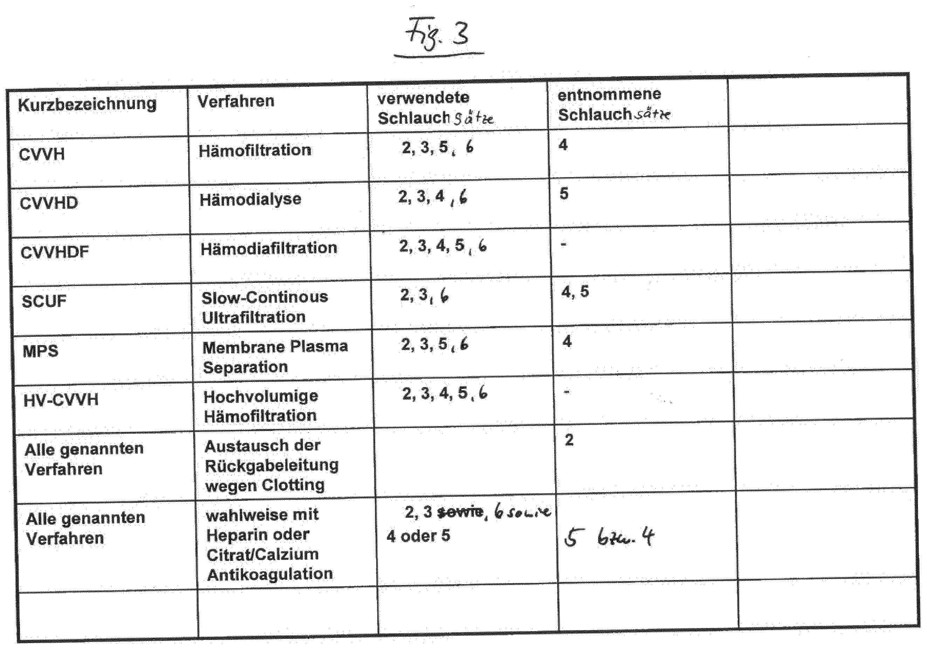

**Fig. 3** gibt in Tabellenform jeweils die für ein bestimmtes Verfahren bzw. eine Behandlungsoption der verwendeten Behandlungsvorrichtung verwendete Komponenten sowie ggf. entnommene Komponenten an.

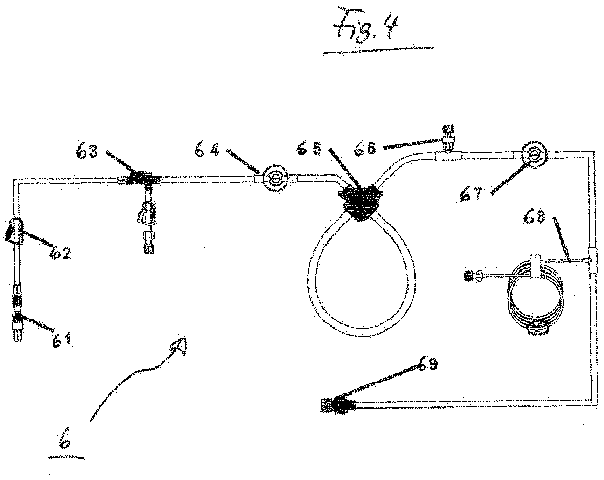

**Fig. 4** zeigt die Zugangsleitung 6 vom Patienten zum Filter, welche auch aus der Fig. 1 bekannt ist. Die Zugangsleitung 6 weist einen Patientenkonnektor 61 mit einer Kappe, eine Schlauchklemme 62, einen Infusionsport 63, eine Druckmessstelle 64, ein Pumpsegment 65 für eine Rollerpumpe, ein Anschlussstück 66 mit Rückschlagventil zum Austausch von Flüssigkeit, eine Druckmessstelle 67 zum Messen eines Vor-Filter-Drucks, eine Anschlussleitung 68 für eine Heparin-Gabe oder eine andere Antikoagulation sowie einen Filteranschluss 69 mit einer Kappe auf.

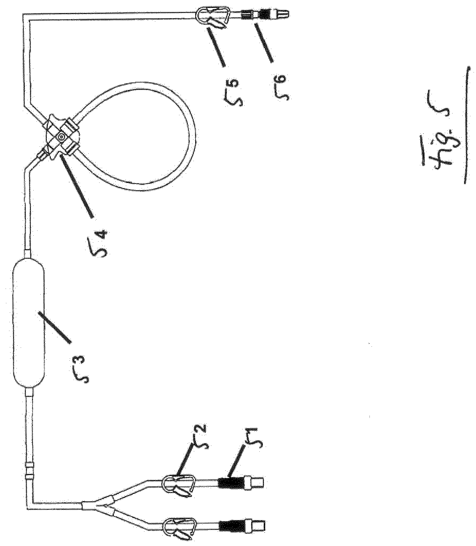

**Fig. 5** zeigt eine Substituatleitung 5, wie sie auch in Fig. 1 gezeigt ist. Die Leitung 5 weist einen Konnektor 51 für einen Lösungsbeutel mit einer Kappe, eine Schlauchklemme 52, einen Heizbeutel 53, ein Pumpsegment 54, eine Schlauchklemme 55 sowie einen Konnektor 56 für ein Leitungs-Anschlussstück mit einer Kappe auf.

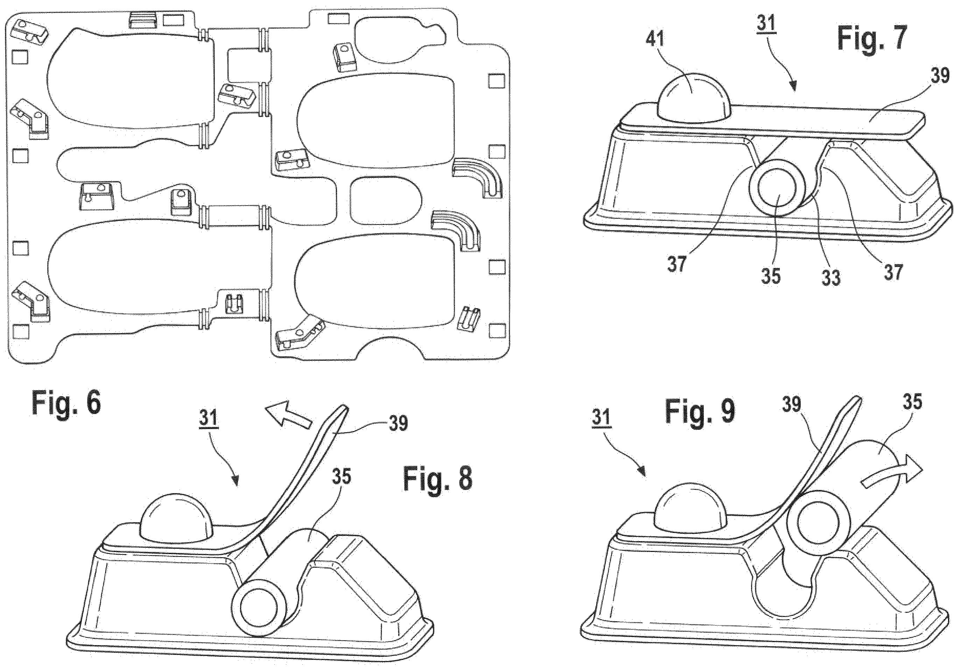

**Fig. 6** zeigt einen nicht mit Blutschlauchelementen bestückten erfindungsgemäßen Organizer 6 im Überblick bzw. in einer Draufsicht.

**Fig. 7** zeigt eine erste Ausführungsform einer Befestigungseinrichtung 31 mit einem Aufnahmeabschnitt 33 zum lösbaren Aufnehmen eines Abschnitts eines Blutschlauchs 35, wobei der Aufnahmeabschnitt 33 geneigte Flächen 37 für eine einfachere Aufnahme des Schlauchabschnitts 35 im Aufnahmeabschnitt 33 aufweist. In Fig. 7 ist ferner eine Lasche 39 gezeigt, welche mittels einer Fixiereinrichtung 41 an der Befestigungseinrichtung 31 fixiert ist. Die Lasche 39 ist, wie in **Fig. 8** zu erkennen ist, elastisch ausgestaltet und lässt sich zur Entnahme des Schlauchs 35 - wie in **Fig. 9** dargestellt - anheben bzw. elastisch hochbiegen. Sie verhindert aufgrund ihrer vorbestimmten Elastizität ein ungewolltes Freigeben des Schlauchs 35 aus dem Aufnahmeabschnitt 33.

Bei der Fixiereinrichtung 41 der Ausführungsformen der Befestigung der Lasche der Figs. 7 bis 9 handelt es sich vorzugsweise um einen Schweißpunkt. In diesem Falle ist die Lasche verdrehsicher mit der Befestigungseinrichtung verbunden. Es ist aber auch möglich, die Fixiereinrichtung 41 verdrehbar auszuführen, z. B. durch einen Niet und/ oder eine drehbare Schnappverbindung. In diesem Falle kann die Lasche durch Verdrehen geöffnet werden, so dass das Schlauchsegment leicht und ohne nennenswerten Kraftaufwand herausgenommen werden kann.

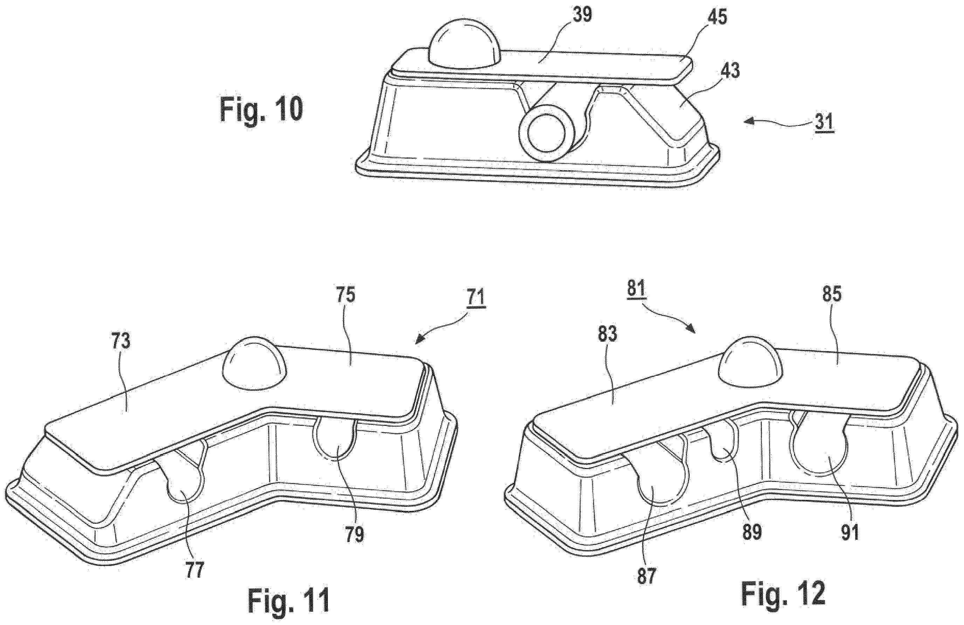

**Fig. 10** zeigt die aus den Figs. 7, 8 und 9 bekannte Befestigungseinrichtung 31, welche einen geneigten Abschnitt 43 aufweist, welcher ein Anheben der Lasche durch den Benutzer begünstigt bzw. vereinfacht. Hierzu trägt auch ein endständiger Rand bzw. Überstand 45 der Lasche 39 bei.

**Fig. 11** zeigt eine Befestigungseinrichtung 71 in einer zweiten Ausführungsform hiervon. Die Befestigungseinrichtung 71 weist zwei Laschen 73 und 75 auf, welche jeweils einen Aufnahmeabschnitt 77 und 79 auf dieselbe Weise wie mit Bezug auf die Fig. 7 bis 10 beschrieben verschließen.

**Fig. 12** zeigt eine Befestigungseinrichtung 81, welche zwei Laschen 83 und 85 aufweist zum Verschließen von drei Aufnahmeabschnitten 87, 89 und 91. Die Aufnahmeabschnitte können bei jeder Ausführungsform jeweils dieselbe oder unterschiedliche geometrische Erstreckungen haben.

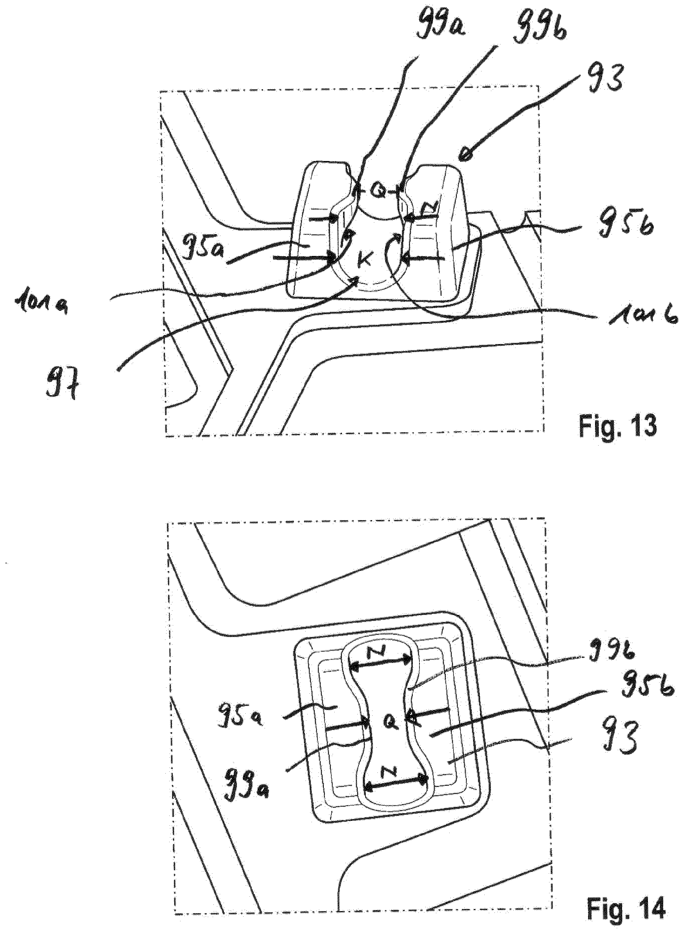

**Fig. 13** zeigt eine erfindungsgemäße Befestigungseinrichtung 93 gemäß einer vierten Ausführungsform in einer perspektivischen Ansicht. Die Befestigungseinrichtung 93 ist als Clip ausgestaltet. Sie weist zwei Schenkel 95a und 95b auf, welche jeweils einen (in Fig. 13) nach oben sowie an seinen Stirnseiten offenen Kanal 97 seitlich begrenzen. Der somit teil- oder halboffen ausgestaltete Kanal 97 dient der Aufnahme eines in Fig. 13 nicht dargestellten Schlauchsegments.

Die Schenkel 95a und 95b haben jeweils einen in Richtung der oberen Öffnung mit der Weite Q des Kanals 97 weisende Vorsprung bzw. eine Verstärkung 99a bzw. 99b. Mittels der Verstärkungen 99a und 99b kann eine Klemmwirkung auf ein in die Befestigungseinrichtung 93 eingelegtes Schlauchsegment (nicht gezeigt) erzielt werden. Es ist auch möglich, die Vorsprünge oder Verstärkungen entlang des Kanals 97 abwechselnd ("auf Lücke") auf der einen oder der anderen Seite des Kanals 97 vorzusehen, derart, dass keine gegenüber liegenden Vorsprünge oder Verstärkungen auftreten oder sich Vorsprünge oder Verstärkungen nicht über den Kanal hinweg gegenüber liegen. Dadurch wird ein besonders bequemes Einlegen des Schlauchs ermöglicht. Ferner ermöglicht dies vorteilhaft, auf einfache und dennoch sichere Weise das Schlauchsegment in der Befestigungseinrichtung 93 lösbar zu befestigen.

Die Ausgestaltung der Befestigungseinrichtung 93 muss zum einfachen und dennoch sicheren Befestigen des Schlauchsegments im Kanal 97 nicht die in den Fig. 13 bis 16 erkennbare Ausgestaltung des Kanalquerschnitts mit Hinterschneidungen 101a und 101b haben. Zwar bieten die Hinterschneidungen 101a und 101b, welche die Weite Q einer Öffnung festlegen, welcher geringer als eine Weite K eines Kanalabschnitts, in welchen das Schlauchsegment innerhalb der Befestigungseinrichtung 93 zum Liegen kommt, eine nochmals erhöhte Sicherheit dafür, dass sich das Schlauchsegment nicht unbeabsichtigt aus der Befestigungseinrichtung 93 löst. Dennoch ist es erfindungsgemäß nicht erforderlich, derartige Hinterschneidung vorzusehen.

Von der Erfindung ebenfalls umfasst ist das Vorsehen von nur einer Hinterschneidung, z.B. 101a oder 101b, an nur einem Schenkel 95a oder 95b. Ebenso ist von der Erfindung umfasst, dass einer der Schenkel 95a oder 95b - oder beide - mehr als nur einen Vorsprung bzw. Verstärkung - wie beispielsweise die Verstärkung 99a oder 99b - aufweist. Auch können die Schenkel 95a oder 95b voneinander abweichende Anzahlen von Vorsprüngen oder Verstärkungen ausweisen (z.B. 0 und 1, 1 und 2, 0 und 2, usw.).

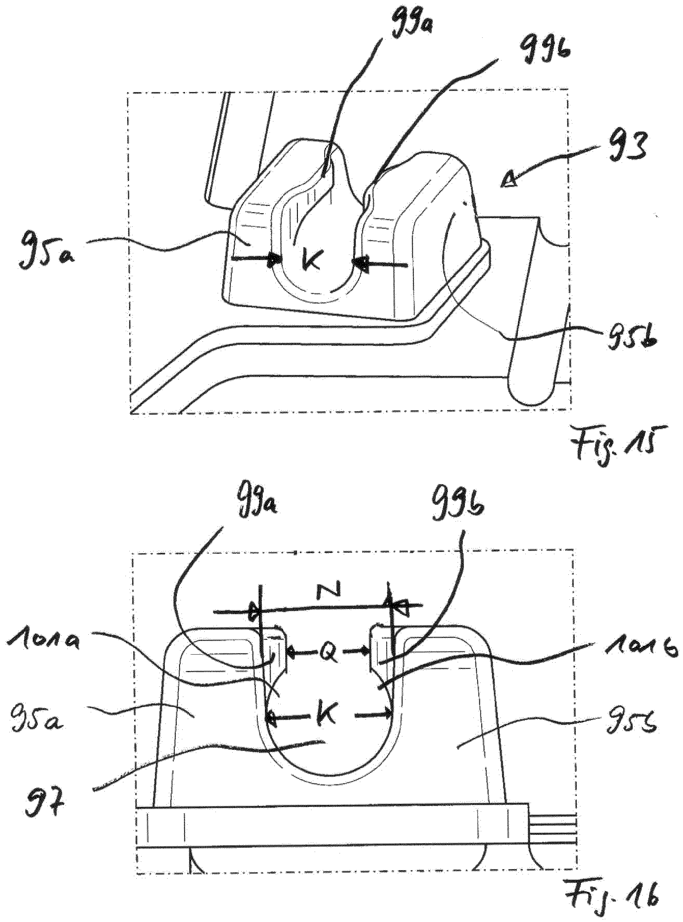

**Fig. 14** zeigt die Befestigungseinrichtung der Fig. 13 in einer Draufsicht. Gut zu erkennen ist, dass die Befestigungseinrichtung 93 aufgrund der Verstärkungen 99a und 99b in der Draufsicht eine Sanduhr-artige Form aufweist. Diese Form weist der Kanal 97 in Längsschnitten in einem oberen Bereich hiervon auf, wie z.B. mit Blick auf die Fig. 15 und besonders die Fig. 16 erkennbar ist. Dort ist die Weite Q geringer als eine Weite N. In einem unteren Bereich der Befestigungseinrichtung 93 (siehe z.B. Fig. 16) kann ein Längsschnitt im Gegensatz hierzu wieder einen rechteckigen Längssschnitt annehmen. Es wird angemerkt, dass die Vorsprünge oder Verstärkungen 99a und 99b nur exemplarisch in einem mittleren Längsabschnitt des Kanals 97 angeordnet sind wie in den Fig. 13 bis 16, was zu der typischen Sanduhrform mancher Längsschnitte führt. Die Vorsprünge oder Verstärkungen 99a und 99b können natürlich auch in einem Bereich nahe einer oder beider Stirnseiten des Kanals 97 und/oder an einer wiederum anderen Stelle vorgesehen sein.

**Fig. 15** zeigt die Befestigungseinrichtung der Fig. 13 und 14 in einer weiteren perspektivischen Ansicht und **Fig. 16** zeigt die Befestigungseinrichtung der Fig. 13 bis 15 in einer Seitenansicht.

Es ist erfindungsgemäß möglich, den erfindungsgemäßen Organizer nach der Behandlung zusammen mit Teilen der Disposables bzw. Komponenten zu entsorgen oder aber den Organizer ohne Disposables einer Wiederverwertung (stoffliches Recycling) zuzuführen. Es ist aber auch möglich, den Organizer einer Wiederverwendung (erneuter Einsatz) zuzuführen. Dazu kann es von Vorteil sein, wenn die Befestigungseinrichtung (z. B. die Lasche 39 oder sogar die komplette Befestigungseinrichtung 31) zusammen mit dem oder den Disposables vom Organizer entfernt und entsorgt werden kann. Als mögliche Ausführungsform der Befestigungseinrichtung ist z. B. die Einrastung mittels Schnappverbindung angedacht. Zur Wiederverwendung des Organizers würde die Lasche/ Befestigungseinrichtung dann durch eine neue Lasche/ Befestigungseinrichtung ersetzt werden.

## Patentansprüche

1. Organizer (1) zum lösbaren Aufnehmen von Komponenten (2, 3, 4, 5, 6, 35) von Blutschlauchsätzen für unterschiedliche extrakorporale Blutbehandlungsoptionen, insbesondere bei Dialyseverfahren, mittels einer Blutbehandlungsvorrichtung (17), **dadurch gekennzeichnet, dass** der Organizer (1) geeignet und vorbereitet ist, Komponenten (2, 3, 4, 5, 6, 35) aufzunehmen, mittels welcher im Gebrauch des Organizers (1) wahlweise wenigstens ein erster Blutschlauchsatz für eine erste Blutbehandlungsoption oder ein zweiter Blutschlauchsatz für eine zweite Blutbehandlungsoption aus einer Gruppe mit wenigstens zwei Blutschlauchsätzen ausgestaltbar sind.

2. Organizer (1) nach Anspruch 1, welcher einstückig ausgestaltet ist.

3. Organizer (1) nach einem der Ansprüche 1 oder 2 mit wenigstens einer Befestigungseinrichtung (31, 71, 81, 93) zum lösbaren Befestigen von Komponenten (2, 3, 4, 5, 6, 35) von Blutschlauchsätzen, insbesondere zum individuellen Lösen von Komponenten (2, 3, 4, 5, 6, 35).

4. Organizer (1) nach Anspruch 3, bei welchem die Befestigungseinrichtung (31, 71, 81) aufweist:
wenigstens einen Aufnahmeabschnitt (33, 77, 79, 87, 89, 91) zum lösbaren Aufnehmen wenigstens einer Komponente (2, 3, 4, 5, 6, 35) am Organizer (1), insbesondere mit wenigstens einer Vertiefung zum Aufnehmen der Komponente (2, 3, 4, 5, 6, 35) entlang eines Abschnitts eines Umfangs der Komponente (2, 3, 4, 5, 6, 35); und
wenigstens eine Lasche (39, 73, 75, 83, 85), mittels welcher die Komponente (35) im Aufnahmeabschnitt (33, 77, 79, 87, 89, 91) lösbar sicherbar ist, insbesondere zum Verschließen der Vertiefung des Aufnahmeabschnitts (33, 77, 79, 87, 89, 91).

5. Organizer (1) nach Anspruch 4, bei welchem die Lasche (39, 73, 75, 83, 85) elastisch ausgestaltet ist.

6. Organizer (1) nach einem der Ansprüche 4 oder 5, wobei die Befestigungseinrichtung (31, 71, 81) wenigstens einen Abschnitt (43) aufweist, welcher derart ausgestaltet ist, dass das Anheben der Lasche (39, 73, 75, 83, 85) mittels des Fingers eines Benutzers erleichtert ist.

7. Organizer (1) nach Anspruch 6, wobei der Abschnitt (43) eine Neigung, insbesondere zu einer flächigen Erstreckung des Organizers (1) aufweist.

8. Organizer (1) nach einem der Ansprüche 4 bis 7, wobei wenigstens ein Abschnitt der Lasche (39, 73, 75, 83, 85) verschwenkbar angeordnet ist.

9. Organizer (1) nach einem der Ansprüche 4 bis 8, wobei die Lasche (39, 73, 75, 83, 85) einen Überstand (45) aufweist zum Anheben oder Verschwenken der Lasche (39, 73, 75, 83, 85) mittels eines Fingers.

10. Organizer (1) nach einem der vorangegangenen Ansprüche, welcher Komponenten (2, 3, 4, 5, 6, 35) zur Ausgestaltung wenigstens eines Blutschlauchsatzes aufweist.

11. Organizer (1) nach Anspruch 10, wobei wenigstens eine Befestigungseinrichtung (31, 71, 81) und wenigstens eine Blutschlauchsatzkomponente (35) derart aufeinander abgestimmt sind, dass die Blutschlauchsatzkomponente (35) in der Befestigungseinrichtung (31, 71, 81, 93) verklemmbar ist.

12. Organizer (1) nach Anspruch 3, 10 oder 11, wobei die Befestigungseinrichtung (93) eine, zwei oder mehr Vorsprünge (99a, 99b) aufweist zum Verengen einer Weite eines Kanals (97) zum Aufnehmen eines Schlauchsegments zu dessen lösbarer Befestigung im Kanal (97), vorzugsweise mittels Verklemmens.

13. Organizer (1) nach Anspruch 12, wobei der eine oder die mehreren Vorsprünge (99a, 99b) Hinterschneidung in einem Inneren des Kanals (97) bilden.

14. Verfahren zum Herstellen eines Organizers (1), insbesondere nach einem der vorangegangenen Ansprüche, **gekennzeichnet durch den Schritt**
Vorbereiten des Organizers (1) zum Aufnehmen von Komponenten (2, 3, 4, 5, 6, 35), mittels welcher im Gebrauch des Organizers (1) wahlweise wenigstens ein erster Blutschlauchsatz für eine erste Blutbehandlungsoption oder ein zweiter Blutschlauchsatz für eine zweite Blutbehandlungsoption aus einer Gruppe mit wenigstens zwei Blutschlauchsätzen ausgestaltbar sind.

15. Verfahren zum Herstellen eines Organizers (1) nach Anspruch 14, **gekennzeichnet durch** den Schritt
Anordnen von Komponenten (2, 3, 4, 5, 6, 35) zur Ausgestaltung von wenigstens einem Blutschlauchsatz aus einer Gruppe mit wenigstens zwei Blutschlauchsätzen am Organizer (1).
